(19) Europäisches Patentamt
European Patent Office
Office européen des brevets

(11) Publication number: **0 103 390**
**A2**

(12) # EUROPEAN PATENT APPLICATION

(21) Application number: **83304506.5**

(22) Date of filing: **04.08.83**

(51) Int. Cl.³: **A 61 K 31/425,** A 61 K 31/41, A 61 K 31/415, A 61 K 31/505 // C07D231/16, C07D231/38, C07D231/40, C07D249/06, C07D277/48, C07D239/46

(30) Priority: **13.08.82 GB 8223303**

(43) Date of publication of application: **21.03.84 Bulletin 84/12**

(84) Designated Contracting States: **BE CH DE FR GB IT LI LU NL SE**

(71) Applicant: **IMPERIAL CHEMICAL INDUSTRIES PLC, Imperial Chemical House Millbank, London SW1P 3JF (GB)**

(72) Inventor: **Wardleworth, James Michael, 43 Ravenswood Road, Wilmslow SK9 6HL Cheshire (GB)**

(74) Representative: **Brown, Ivor James Stewart et al, Imperial Chemical Industries PLC Legal Department: Patents PO Box 6, Welwyn Garden City Herts, AL7 1HD (GB)**

(54) **Guanidine-containing esters as histamine H2 receptor blockers.**

(57) Pharmaceutical compositions of ester intermediates of the formula I:

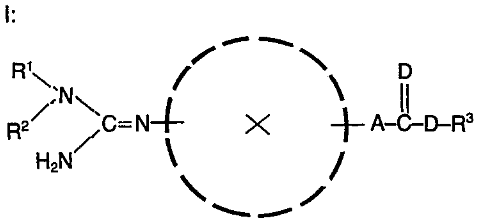

described in European Patent Publication 60094. The compositions have histamine H-2 receptor blocking activity.

EP 0 103 390 A2

ESTERS

TITLE MODIFIED
see front page

This invention relates to esters which have histamine H-2 receptor blocking activity

In European Patent Publication 60094 there are described and claimed certain ester derivatives which are intermediates for the manufacture of the corresponding carboxamide derivatives, these latter compounds being potent histamine H-2 receptor blocking agents. It has now surprisingly been discovered that the ester intermediates themselves have potent histamine H-2 receptor blocking activity.

According to the invention there is provided a pharmaceutical composition which comprises a guanidine derivative of the formula I:-

[Formula I given hereafter]

in which $R^1$ and $R^2$, which may be the same or different, are hydrogen or branched or unbranched 1-10C alkyl, 3-8C cycloalkyl or 4-14C cycloalkylalkyl, each alkyl, cycloalkyl or cycloalkylalkyl being optionally substituted by one or more halogens selected from fluorine, chlorine and bromine, provided that at least one of $R^1$ and $R^2$ is halogen-substituted alkyl, cycloalkyl or cycloalkylalkyl and provided there is no halogen substituent on the carbon atom of the alkyl, cycloalkyl or cycloalkylalkyl which is directly attached to the nitrogen, or $-R^2$ is hydrogen and $-R^1$ is a radical of the formula II:-

[Formula II]

in which W is an unbranched 2-6C alkylene chain which is optionally substituted by one or two 1-4C alkyls, E is oxygen, sulphur, sulphinyl or sulphonyl or a radical of the formula $NR^5$ in which $R^5$ is hydrogen or 1-6C alkyl, $R^4$ is hydrogen or an unbranched 1-6C alkyl which is optionally substituted by one or two 1-4C alkyls, or $R^4$ and $R^5$ are joined to form, together with the nitrogen to which they are attached, pyrrolidine, piperidine, morpholine, piperazine or N-methylpiperazine;

ring X is a phenyl ring or a ring of the formula III:-

[Formula III]

in which the dotted line is a double bond on one side of the nitrogen and Z is carbon or nitrogen such that ring X is a 5- or 6-membered heterocyclic aromatic ring which contains at least one nitrogen and may optionally contain one or two additional hetero atoms selected from oxygen, nitrogen and sulphur, ring X, where possible, carrying one or two optional substituents selected from fluorine, chlorine, bromine, 1-6C alkyl, 1-6C alkoxy, 1-6C alkylthio, trifluoromethyl, hydroxy and amino;

A is phenylene or 5-7C cycloalkylene, or a 1-8C alkylene chain which is optionally substituted by one or two 1-3C alkyls and into which is optionally inserted, as part of the backbone of the chain, one or two groups selected from oxygen, sulphur, NH, 1-6C N-alkyl, <u>cis</u> and <u>trans</u> vinylene, ethynylene, phenylene and 5-7C cycloalkylene, provided that the shortest link between ring X and C=D is of at least 3 atoms, provided that when an optional insertion is made in chain A which results in the inserted group being directly attached to C=D the inserted group is other than oxygen, sulphur, NH or N-alkyl, and provided that no two insertions selected from

oxygen, sulphur, NH and N-alkyl are directly attached one to the other;
D is oxygen or sulphur;
$R^3$ is 1-6C alkyl, phenyl or benzyl;

and the pharmaceutically-acceptable acid addition salts thereof in association with a pharmaceutically-acceptable diluent or carrier.

It is to be understood that, in the above formula I and throughout this specification, although the double bond in the guanidine residue attached to ring X has been inserted in a particular position, other tautomeric forms are possible, and this invention includes such tautomeric forms within its scope. It is also to be understood that when A is or contains cycloalkylene the groups attached to this radical may be in the cis or trans configuration. When A is or contains cycloalkylene and/or when A is an alkylene chain substituted by one or two alkyls, the compound of the formula I will, in most instances, contain at least one asymmetric centre. In such cases the compound of the formula I will therefore exist in at least two enantiomeric forms, the precise number being determined by the number of asymmetric centres. The biological activity, as hereinafter defined, of these enantiomeric forms may differ, and it is therefore to be understood that this invention encompasses the racemate of the formula I, including any possible diastereoisomeric forms, and any enantiomeric form which possesses the disclosed biological activity, it being a matter of common general knowledge to one skilled in the art how to separate diastereoisomeric forms and how to separate a racemate into its enantiomers and determine

the biological activity of each. When ring X is of the formula III it is to be understood that A is attached to Z.

A particular value for $R^1$ and $R^2$ when it is halogen-substituted alkyl is 2,2,2-trifluoroethyl, 2,2,2-trichloroethyl, 2-chloro-2,2-difluoroethyl, 2,2-dichloro-2-fluoroethyl, 2-bromo-2,2-difluoroethyl, 2,2-dibromo-2-fluoroethyl, 2-fluoroethyl, 2-chloroethyl, 2,2-difluoroethyl, 2,2-dichloroethyl, 2-chloro-2-fluoroethyl, 2-bromo-2-fluoroethyl, 2,2,3,3-tetrafluoropropyl, 2,2,3,3,3-pentafluoropropyl, 1,1,1,3,3,3-hexafluoroisopropyl, 1,3-dichloro-1,1,3,3-tetrafluoroisopropyl, 1-chloro-1,1,3,3,3-pentafluoroisopropyl, 1,3-difluoroisopropyl or 2,2,3,3,4,4,4-heptafluorobutyl.

A particular value for $R^1$ or $R^2$ when it is halogen-substituted cycloalkyl is 2,2,3,3-tetrafluorocyclopropyl, 2-chloro-2,3,3-trifluorocyclopropyl, 2,2-difluorocyclopropyl, 2-chloro-3,3-difluorocyclopropyl, 2,2,3,3,4,4-hexafluorocyclobutyl or 2-chloro-2,3,3,4,4-pentafluorocyclobutyl.

A particular value for $R^1$ or $R^2$ when it is halogen-substituted cycloalkylalkyl is (1,2,2,3,3-pentafluorocyclopropyl)methyl, (2-chloro-1,2,3,3-tetrafluorocyclopropyl)methyl, (1,2,2,3,3,4,4-heptafluorocyclobutyl)methyl or (2-chloro-1,2,3,3,4,4-hexafluorocyclobutyl)methyl.

A particular value for $R^1$ or $R^2$ when it is alkyl is methyl, ethyl, propyl, isopropyl or butyl.

A particular value for $R^1$ or $R^2$ when it is cycloalkyl is cyclopropyl or cyclobutyl.

A particular value for $R^1$ or $R^2$ when it is cycloalkylalkyl is cyclopropylmethyl or cyclobutylmethyl.

A particular value for the optional substituent on W is methyl.

A particular value for $R^4$ or $R^5$ is hydrogen or methyl.

A particular value for the radical of the formula II is 2-methoxyethyl, 2-hydroxyethyl, 5-hydroxypentyl, 2-methylthioethyl or 2-dimethylaminoethyl.

A particular value for ring X is a phenyl, oxazole, thiazole, imidazole, 1,2,4-thiadiazole, 1,2,4-oxadiazole, 1,2,3-triazole, 1,2,4-triazole, pyrazole, pyrazine, pyridine, pyrimidine or 1,3,5-triazine ring.

A particular value for the optional substituent on ring X when it is alkyl, alkoxy or alkylthio is methyl, methoxy or methylthio.

A particular value for -A- is phenylene, cyclopentylene, cyclohexylene, trimethylene, tetramethylene, pentamethylene, thioethylene, thiotrimethylene, thiotetramethylene, thiopentamethylene, oxyethylene, oxytrimethylene, oxytetramethylene, methylenethiomethylene, methylenethioethylene, methylenethiopropylene, methyleneoxymethylene, methyleneoxyethylene, ethyleneoxyethylene, oxy-2-methylethylene, thiopropylenethiomethylene, oxyethyleneoxymethylene, iminoethylene, iminopropylene, vinylenepropylene, oxymethylenevinylene, 1,3-phenylene, 1,3-cyclopentylene, methylene-1,4-phenylene, ethyleneoxymethylene-1,4-phenylene, oxy-1,3-phenylenemethylene or thiomethyleneethynylenemethylene. These values for -A- are written reading from left to right in formula I such that the first named part of the radical is attached to ring X and the last named part of the radical is attached to C=D. Thus, for example, when -A- is a methylenethioethylene the compound of the formula I contains the part structure IV:-

[Formula IV]

A particular value for $R^3$ is methyl, ethyl, phenyl or benzyl.

The preferred composition of the invention is one containing methyl 5-[3-(2-[2,2,2-trifluoroethyl]guanidino)pyrazol-1-yl]valerate and the pharmaceutically-acceptable acid-addition salts thereof.

A suitable pharmaceutically-acceptable acid-addition salt of the guanidine deivative of the formula I is, for example, a salt formed with hydrochloric, hydrobromic, phosphoric, sulphuric, acetic, citric or maleic acid.

The pharmaceutical composition of the invention may, for example, be in a form suitable for oral, rectal, parenteral or topical administration, for which purposes it may be formulated by means known to the art into the form of, for example, tablets, capsules, aqueous or oil solutions or suspensions, emulsions, dispersible powders, suppositories, sterile injectable aqueous or oily solutions or suspensions, gels, creams, ointments or lotions.

An example of a tablet formulation is given in Example 1. It will be recognised by those skilled in the art that the method of preparation of this formulation represents only one particular method of preparing such a formulation, and for example the amount of active agent and the amount and/or nature of the excipients may be varied to satisfy particular requirements.

In addition to the guanidine derivative of the formula I, the pharmaceutical composition of the invention for oral, rectal or parenteral administration may also contain, or be co-administered with, one or more known drugs selected from antacids, for example aluminium hydroxide - magnesium hydroxide mixtures; antipepsin compounds, for example pepstatin; other histamine H-2 antagonists, for example cimetidine or ranitidine; ulcer healing agents, for example carbenoxolone or bismuth salts; anti-inflammatory agents, for example ibuprofen, indomethacin, naproxen or aspirin; prostaglandins, for example 16,16-dimethylprostaglandin $E_2$; classical anti-histamines (histamine H-1 antagonists), for example mepyramine or diphenhydramine; anticholinergic agents, for example atropine or propantheline bromide; anxiolytic agents, for example diazepam, chlordiazepoxide or phenobarbital.

The pharmaceutical composition of the invention for topical administration may also contain, in addition to the guanidine derivative, one or more classical anti-histamines (histamine H-1 antagonists), for example mepyramine or diphenhydramine and/or one or more steroidal anti-inflammatory agents, for example fluocinolone or triamcinolone.

A topical formulation may contain 1-10% w/w of the guanidine derivative.

A preferred pharmaceutical composition of the invention is one suitable for oral administration in unit dosage form, for example a tablet or capsule which contains between 5 mg. and 500 mg. of the guanidine derivative, or one suitable for intravenous, subcutaneous or intramuscular injection, for example a sterile injectable containing between 0.1% and 10% w/w of the guanidine derivative.

The pharmaceutical composition of the invention will normally be administered to man for the treatment of peptic ulcers and other conditions caused or exacerbated by gastric acidity in the same general manner as that employed for cimetidine, due allowance being made in terms of dose levels for the potency and duration of action of the guanidine derivative of the present invention relative to cimetidine. Thus according to a further feature of the invention there is provided a method of inhibiting gastric acid secretion in a warm-blooded animal which comprises administering to the animal an effective amount of the compound of the formula I, or a pharmaceutically-acceptable acid-addition salt thereof. Each patient will receive an oral dose of between 15 mg. and 1500 mg., and preferably between 20 mg. and 200 mg., of guanidine derivative or an intravenous, subcutaneous or intramuscular dose of between 0.5 mg. and 50 mg., and preferably between 2 mg. and 20 mg., of the guanidine derivative, the composition being administered 1 to 4 times per day. The rectal dose will be approximately the same as the oral dose. The composition may be administered less frequently when it contains an amount of guanidine derivative which is a multiple of the amount which is effective when given 1-4 times per day.

The guanidine derivative contained in the composition of the invention may be manufactured by methods in which the actual chemical reactions involved are known in themselves. The following processes, $R^1$, $R^2$, $R^3$, A, D and ring X having the meanings stated above, unless indicated otherwise, are illustrative.

(a) construction of the guanidine attached to ring X by reaction of the appropriate thiourea, or a 1-6C S-alkyl (e.g. S-methyl) or S-benzyl derivative thereof, or a salt of such a derivative, with the appropriate amine. The guanidine in the compound of the formula I contains three nitrogens each of which carries different substituents. The appropriate amine for use in this reaction may therefore be ammonia, an amine of the formula $R^1R^2NH$ or an amine of the formula V:-

[Formula V]

The reaction may be conducted using an excess of one of the reactants as a diluent or solvent, or an additional diluent or solvent, for example methanol or ethanol, may be added. In many cases it is advantageous to use a catalyst such as mercuric oxide, lead oxide or sodium hypochlorite. The reaction may be conducted at ambient temperature or it may be accelerated or completed by the application of heat, for example by heating to the boiling point of the diluent or solvent.

(b) construction of the guanidine attached to ring X by reaction of the appropriate cyanamide with the appropriate amine. Since the guanidine in the compound of the formula I contains only one unsubstituted nitrogen there are two appropriate amines, namely the amine of the formula $R^1R^2NH$ or of the formula V given above.

(c) for those compounds in which the group inserted into A is oxygen, sulphur, NH or N-alkyl, reaction of a compound of the fomula VI or VII

[Formula VI]

[Formula VII]

with a compound of the formula VIII or IX respectively:-

[Formula VIII]

[Formula IX]

in which G is oxygen, sulphur, NH or N-alkyl, $R^6$ is a displaceable radical and $A^1$ and $A^2$ are fragments of A, including direct bonds, and are such that $A^1$-G-$A^2$ falls within the definition of A given above. $R^6$ is, for example, halogen, for example chlorine, bromine or iodine. When $R^6$ is directly attached to ring X, $R^6$ may, for example, be methylsulphinyl or methylsulphonyl.

(d) for those compounds in which in formula III Z is nitrogen, reaction of a compound of the formula X:-

[Formula X]

with a compound of the formula XI:-

[Formula XI]

in which $R^6$ is a displaceable radical. $R^6$ is, for example, halogen, for example chlorine, bromine or iodine.

(e) for those compound in which ring X is a thiazole ring, reaction of a compound of the formula XII:-

[Formula XII]

with a compound of the formula XIII:-

[Formula XIII]

in which Hal is chlorine or bromine and $R^7$ is hydrogen or the optional substituent on the thiazole ring. The reaction may be conducted in a diluent or solvent such as acetone and may be accelerated or completed by the appliction of heat, for example by heating to the boiling point of the diluent or solvent.

(f) reaction of a compound of the formula XIV:-

[Formula XIV]

or an activated derivative (for example halide, anhydride) thereof with a compound of the formula $R^3DH$. When the compound of the formula XIII is used as such the reaction may be conducted in the presence of a catalyst such as sulphuric acid.

(g) reaction of a compound of the formula XV with a compound of the formula $R^3DH$ in the presence of a catalyst (e.g. sulphuric acid).

When the process manufactures the compound of the formula I in the form of the free base and an acid-addition salt is required, the compound of the formula I in the free base form is reacted with an acid which affords a pharmaceutically-acceptable anion.

The starting material of the formula XIV for use in process (f) may be obtained by separate construction of the two side chains on the appropriate ring X. Thus the left hand side chain may be constructed by reduction of a nitro group to an amino group, reaction of this amino group with an isothiocyanate of the formula $R^1$-N=C=S, and finally reaction of the resulting thiourea with ammonia in the presence of mercuric oxide. The method of construction of the

right hand side chain may vary depending on the nature of ring X, the nature of the atom in ring X to which A is attached (carbon or nitrogen) and the presence or absence of inserted atoms or groups in chain A. In this construction it may be necessary to protect the acid function as a cyano group and to hydrolyse to the acid as a final step. When A contains no inserted group or the inserted group is phenylene and Z is a carbon, it is preferable to construct the ring X with the right hand chain already in place. Thus when ring X is a thiazole ring a process similar to that described in process (e) may be used. When ring X is a 1,2,3-triazole ring, it may be formed by reaction of methazonic acid with a suitable azide. When ring X is a pyrimidine, it may be formed by reaction of a suitably substituted imino ether with 2-chloroacrylonitrile. When the inserted group in A is vinylene or ethynylene, A may be introduced by formation of the double or triple bond by standard coupling methods. When the inserted group in A is cycloalkylene radical, the chain A may be constructed by a conjugate addition to the corresponding cycloalk-2-enone. When the inserted group in A is oxygen, sulphur, NH or N-alkyl, the right hand chain maybe built up by a method similar to that described in process (c). When Z is nitrogen, the right hand chain may be formed by a method similar to that described in process (d).

The starting material of the formula V for use in process (a) may be prepared by the methods described above for the preparation of the compounds of the formula XIV in which the right hand chain is constructed first.

The cyanamide, corresponding to the amine of the formula V, for use in process (b) may be prepared by reaction of the compound of the formula V with cyanogen bromide.

The starting materials of the formulae VI and VIII for use in process (c), and of the formula X for use in process (d), may be prepared by construction of the guanidine chain on a suitably substituted ring X.

As noted above, the guanidine derivative contained in the composition of the invention is a histamine H-2 antagonist, inhibits the secretion of gastric acid in warm-blooded animals and is therefore useful in the treatment of peptic ulcers and other conditions caused or exacerbated by gastric acidity, including stress ulcers and gastrointestinal bleeding due to trauma.

The histamine H-2 antagonist activity may be demonstrated on standard tests, for example by the ability of the compound of the formula I to innhibit the histamine-induced positive chronotropic response in the spontaneously beating right atrium of the guinea pig or by its ability to inhibit the histamine-induced uptake of aminopyrine into the acid space of parietal cells.

All the compounds exemplified in this specification were tested either on the guinea pig atrium test or on the aminopyrine test. All those tested on the guinea pig atrium test are active at or below a bath concentration of 10 μM. and the more active compounds show complete inhibition of response at this concentration. All those tested on the aminopyrine test gave a 50% inhibition of uptake of aminopyrine at or below a concentration of 3 μM.

The inhibition of the secretion of gastric acid maybe demonstrated in standard tests, for example by the ability of the compound of the fomula I, when dosed intravenously, intragastically or orally,

to inhibit the secretion of acidic gastric juice in, for example, rats or dogs provided with gastric fistulae or denervated fundic pouches, and whose gastric secretion is stimulated by administration of a secretagogue, for example histamine, pentagastrin, bethanechol or food.

The results obtained in the atrium and aminopyrine tests are predictive of activity in the rat and dog tests. No overt toxicity or side effects were noted during the rat or dog tests.

The preferred compound exemplified in this specification exhibits inhibition of acid secretion which shows little or no decline from peak inhibition for several hours.

The N-methylcyanoguanidine group in known H-2 receptor antagonists is potentially changeable into the mutagenic N-nitroso N-methylcyanoguanidine group in the mammalian body (Pool et al., Toxicology, 1979, 15, 69). The corresponding group in the compounds of the present invention, $CDDR^3$, is not potentially changeable into carcinogenic nitroso derivatives.

The invention is illustrated, but not limited, by the following Examples. The n.m.r. spectra are quoted in $\delta$ relative to tetramethylsilane ($\delta$ =0) as internal standard (s = singlet, d = doublet, t = triplet, q = quartet, m = multiplet, br = broad). The temperatures are in degrees Centigrade. The following contractions are used:-

| | | |
|---|---|---|
| DMF | = | dimethyl formamide |
| DMSO | = | dimethyl sulphoxide |
| MeOH | = | methanol |
| EtOH | = | ethanol |
| THF | = | tetrahydrofuran |
| HOAc | = | acetic acid |
| EtOAc | = | ethyl acetate |

Attention is drawn to the fact that 3-nitropyrazole (Example 1) is an explosion hazard.

Example 1

A tablet containing 50 mg. of methyl 5-[3-(2-[2,2,2-trifluoroethyl]guanidino)pyrazol-1-yl]valerate may be prepared using ingredients in the following proportions:-

| | | |
|---|---|---|
| (a) | Tablet Core | mg./tablet |
| | Active agent | 50 |
| | Lactose | 218.5 |
| | Calcium carboxymethylcellulose | 22.5 |
| | Polyvinylpyrrolidone | 6.0 |
| | Magnesium Stearate | 3.0 |
| (b) | Tablet Coat | mg./tablet |
| | Hydroxypropylmethylcellulose | 4.5 |
| | Polyethylene glycol | 0.9 |
| | Titanium dioxide | 1.35 |

The active agent, lactose and calcium carboxymethylcellulose are mixed. An aqueous solution of polyvinylpyrrolidone is added, and the mass is then mixed until it is suitable for granulation. The mass is then granulated and dried. The magnesium stearate is blended with the dried granules and the resulting mixture is compressed into tablets. The tablets are film-coated using an aqueous or solvent suspension of hydroxypropylmethylcellulose, polyethylene glycol and titanium dioxide.

The active ingredient used in the preparation of the above formulation may be prepared as follows:-

3-Nitropyrazole (10 g.) was stirred in DMF (100 ml.) with sodium hydride (3.89 g. of a 60% w/w paste in mineral oil) for 1 hour. Methyl 5-bromovalerate (19 g.) was added and the mixture was stirred at $25^{o}$ for 20 hours. The mixture was diluted with water (500 ml.) and extracted with EtOAc (3 x 500 ml.). The extract was dried ($MgSO_4$) and evaporated in vacuo to an oil (24 g.) which was fractionated on an m.p.l.c.

silica column (600 m.m. x 38 m.m.) eluted with EtOAc/ petroleum ether (b.p. 60-80°) 1:2 v/v. The major product (14.2 g.) was methyl 5-(3-nitropyrazol-1-yl)valerate and had an $R_f$ value of 0.2 on a silica thin layer plate eluted with EtOAc/petroleum ether (b.p. 60-80°) 1:1 v/v.

The above nitro ester (12 g.) was hydrogenated at atmospheric pressure in molecular sieve-dried THF (150 ml.) over 5% w/w palladium on carbon (1 g.). After hydrogen uptake had ceased the catalyst was filtered off and the filtrate was evaporated *in vacuo*. The residue was crystallised from EtOAc to give methyl 5-(3-aminopyrazol-1-yl)valerate (88%), m.p. 83-84°.

The above amino ester (8 g.) was dissolved in acetonitrile (150 ml.) and 2,2,2-trifluoroethyl isothiocyanate (16 ml.). After two hours volatile material was evaporated *in vacuo* and the residue was crystallised from EtOH to give methyl 5-[3-(3-[2,2,2-trifluoroethyl]-thioureido)pyrazol-1-yl]valerate, m.p. 111°.

The above thiourea ester (0.5 g.) was stirred in 6M ammonia in MeOH (10 ml.) with mercuric oxide (1.2 g.) for 3 hours. The mixture was filtered and the filtrate was evaporated *in vacuo*. The residue was crystallised from toluene to give methyl 5-[3-(2-[2,2,2-trifluoroethyl]guanidino)pyrazol-1-yl]valerate (0.25 g.), m.p. 78°.

Example 2

A tablet containing 50 mg. of active ingredient may be obtained by repeating the directions given in Example 1, using 50 mg. of one of the following active ingredients A-D in place of methyl 5-[3-(2-[2,2,2-trifluoroethyl]guanidino)pyrazol-1-yl]-valerate.

A. methyl 3-[2-(2-[2,2,2-trifluoroethyl]-guanidino)thiazol-4-ylmethylthio]propionate.

B. methyl 5-[2-(2-[2,2,2-trifluoroethyl]guanidino)thiazol-4-yl]valerate.

C. ethyl 4-[4-(2-[2,2,2-trifluoroethyl]guanidino)pyrimid-2-ylthio]butyrate.

D. methyl 3-[4-(2-[2,2,2-trifluoroethyl]guanidino)-1,2,3-triazol-2-yl]benzoate.

The active ingredients A-D may be prepared as follows:-

A. A mixture of 2-[2,2,2-trifluoroethyl]-guanidino-4-chloromethylthiazole hydrochloride (4.6 g.) in EtOH (75 ml.) and methyl 3-mercaptopropionate (2.47 ml.) at $5^{o}$ was treated dropwise with aqueous sodium hydroxide (1.8 g. in 15 ml. of water) over 10 minutes. The resulting solution was allowed to reach room temperature and was stirred for 1 hour. It was then poured into water and the precipitate filtered and crystallised from EtOH to give methyl 3-[2-(2-[2,2,2-trifluoroethyl]guanidino)thiazol-4-ylmethylthio]propionate (1.93 g.), m.p. 96-$98^{o}$.

B. To a solution of methyl 7-chloro-6-oxoheptanoate (2.0 g.) in hot EtOH (20 ml.) was added a solution of (2,2,2-trifluoroethyl)amidinothiourea (2.1 g.) in hot EtOH (20 ml.). The resulting mixture was heated under reflux for 1 hour. The mixture was then evaporated to dryness and the residue partitioned between ether (20 ml.) and water (60 ml.). The aqueous layer was separated and basified with sodium bicarbonate and extracted with EtOAc. The EtOAc solution was evaporated to dryness and the residue crystallised from EtOAc/ether containing a small amount of acetone to give methyl 5-[2-(2-[2,2,2-trifluoroethyl]guanidino)-thiazol-4-yl]valerate having the following n.m.r. spectrum in $d_6$DMSO:- 1.4-1.8 (m, 4H); 2.2-2.7 (m, 4H); 3.6 (s, 3H); 3.8-4.3 (m, 2H); 6.4 (s, 1H).

C. A mixture of 2-thiocytosine (0.64 g.), ethyl 4-bromobutyrate (1.07 g.) and 1,5-diazabicyclo[5,4,0]undec-5-ene (0.84 g.) was stirred for 4 hours and then evaporated to dryness. The residue was treated with water and the mixture extracted with EtOAc, and the extract dried and evaporated to dryness to give a gum (1.9 g.). The gum was dissolved in acetonitrile (5 ml.), the solution treated with 2,2,2-trifluoroethyl isothiocyanate (1.1 g.) and the mixture heated at 70$^{o}$ for 72 hours with the addition of further portions of isothiocyanate (0.5 g.) at 24 and 48 hours. The mixture was cooled and the solid which crystallised was collected to give ethyl 4-[4-(3-[2,2,2-trifluoroethyl]thioureido)-pyrimid-2-ylthio]butyrate, m.p. 104-106$^{o}$.

A mixture of ethyl 4-[4-(3-[2,2,2-trifluoroethylthioureido)pyrimid-2-ylthio]butyrate (0.25 g.), DMF (2 ml.), saturated ethanolic ammonia (5 ml.) and yellow mercuric oxide (0.2 g.) was stirred at room temperature for 2 hours and then filtered. The filtrate was evaporated to dryness and the residue recrystallised from EtOAc to give ethyl 4-[4-(2-[2,2,2-trifluoroethyl]guanidino)pyrimid-2-ylthio]butyrate, m.p. 120-122$^{o}$.

D. Nitromethane (61 g.) was added to a warm (45-50$^{o}$) solution of NaOH (61 g.) in water (122 ml.) at such a rate that the temperature was maintained. At the end of the addition the temperature was raised to 55$^{o}$ for 10 minutes and then allowed to fall back to 50$^{o}$. The mixture was chilled and neutralised to pH 7 with concentrated hydrochloric acid at <10$^{o}$. The precipitated product was redissolved by the addition of aqueous NaOH (12.5N; 40 ml.) to give a solution of the sodium salt of methazonic acid.

A solution of $NaNO_2$ (36.2 g.) in water (300 ml.) was added during approximately 30 minutes to a suspension of 3-aminobenzoic acid (68.6 g.) in concentrated hydrochloric acid (126.3 ml.) and water (200 ml.) at 0-5°. The mixture was filtered to give a solution of 3-carboxybenzenediazonium chloride.

The solution of the sodium salt of methazonic acid was treated at 10° with a cold (5°) solution of 3-carboxybenzenediozonium chloride. A precipitate formed immediately and was dissolved in aqueous NaOH (33% w/w; 100 ml.) to give a dark red solution. The dark red solution was stirred and treated at 25° with acetic anhydride (100 ml.). During this treatment, aqueous NaOH (33% w/w, 200 ml.) was added to keep the mixture basic. The reaction mixture was acidified with concentrated hydrochloric acid and the precipitated product was isolated by filtration to give 101.2 g. of a light brown solid. A mixture of 23.5 g. of this solid, MeOH (150 ml.) and concentrated sulphuric acid (0.5 ml.) was heated under reflux for 3 hours. The reaction mixture was neutralised with aqueous NaOH (1N), concentrated, and partitioned between $CHCl_3$ and brine. The $CHCl_3$ phase was dried ($MgSO_4$) and evaporated to give 5.9 g. of a red oil that crystallised slowly. The solid was purified by medium pressure liquid chromatography on a silica gel column using EtOAc as eluant to give 5.3 g. of a solid which was recrystallised twice from isopropanol to give 2.7 g. of methyl 3-(4-nitro-1,2,3-triazol-2-yl)benzoate, m.p. 104-106°.

A mixture of methyl 3-(4-nitro-1,2,3-triazol-2-yl)benzoate (1.0 g.), 5% w/w palladium on charcoal (0.5 g.) and HOAc (100 ml.) was stirred under one atmosphere of hydrogen until 300 ml. of hydrogen had

been absorbed. The reaction was filtered and evaporated to give 0.91 g. of methyl 3-(4-amino-1,2,3-triazol-2-yl)benzoate, m.p. 132-134° after recrystallisation from MeOH.

A warm mixture of methyl 3-(4-amino-1,2,3-triazol-2-yl)benzoate (0.44 g.) and acetonitrile (5 ml.) was treated with 2,2,2-trifluoroethylisothiocyanate (0.34 g.), allowed to cool, and kept at room temperature for 21 hours. The reaction mixture was filterd, washed with ether and petroleum ether (b.p. 60-80°), and dried to give 0.63 g. of methyl 3-[4-(3-[2,2,2-trifluoroethyl]thioureido)-1,2,3-triazol-2-yl]benzoate, m.p. 187-188°.

A mixture of methyl 3-[4-(3-[2,2,2-trifluoroethyl]thioureido)-1,2,3-triazol-2-yl]benzoate (0.5 g.), mercuric oxide (0.4 g.) and ammoniacal EtOH (6M; 10 ml.) was stirred at room temperature for 2 hours. The mixture was treated with mercuric oxide (0.1 g.) and stired for another 2 hours. The mixture was filtered and evaporated to give 0.47 g. of methyl 3-[4-(2-[2,2,2-trifluoroethyl]guanidino)-1,2,3-triazol-2-yl]benzoate, m.p. 171-173° after recrystallisation from EtOAc and petroleum ether (b.p. 60-80°).

Imperial Chemical Industries PLC
Formulae Drawings
"Esters"

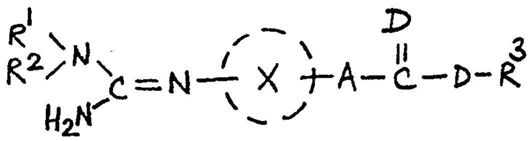

I

$R^4-E-W-$

II

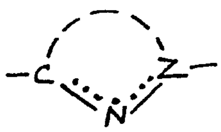

III

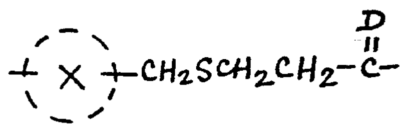

IV

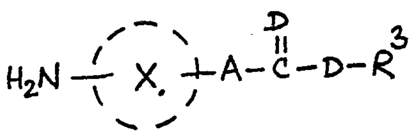

V

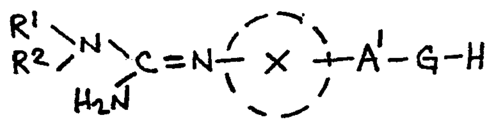

VI

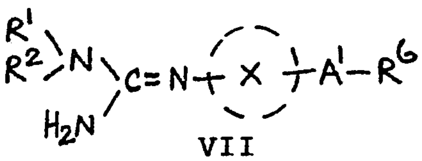

VII

$R^6-A^2-\overset{D}{\overset{\|}{C}}-D-R^3$

VIII

$H-G-A^2-\overset{D}{\overset{\|}{C}}-D-R^3$

IX

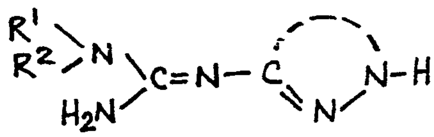

X

$R^6-A-\overset{D}{\overset{\|}{C}}-D-R^3$

XI

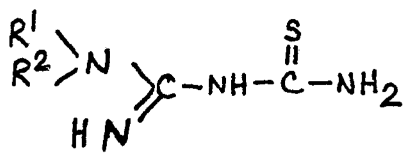

XII

$Hal-\overset{R^7}{\overset{|}{C}}H-CO-A-\overset{D}{\overset{\|}{C}}-D-R^3$

XIII

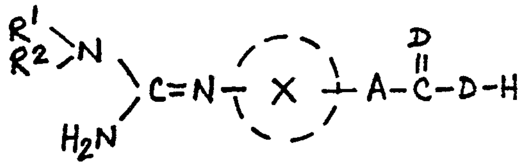

XIV

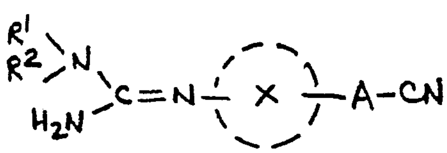

XV

Claims

1. A pharmaceutical composition which comprises a guanidine derivative of the formula I:-

[Formula I]

in which $R^1$ and $R^2$, which may be the same or different, are hydrogen or branched or unbranched 1-10C alkyl, 3-8C cycloalkyl or 4-14C cycloalkylalkyl, each alkyl, cycloalkyl or cycloalkylalkyl being optionally substituted by one or more halogens selected from fluorine, chlorine and bromine, provided that at least one of $R^1$ and $R^2$ is halogen-substituted alkyl, cycloalkyl or cycloalkylalkyl and provided there is no halogen substituent on the carbon atom of the alkyl, cycloalkyl or cycloalkylalkyl which is directly attached to the nitrogen, or $-R^2$ is hydrogen and $-R^1$ is a radical of the formula II:-

[Formula II]

in which W is an unbranched 2-6C alkylene chain which is optionally substituted by one or two 1-4C alkyls, E is oxygen, sulphur, sulphinyl or sulphonyl or a radical of the formula $NR^5$ in which $R^5$ is hydrogen or 1-6C alkyl, $R^4$ is hydrogen or an unbranched 1-6C alkyl which is optionally substituted by one or two 1-4C alkyls, or $R^4$ and $R^5$ are joined to form, together with the nitrogen to which they are attached, pyrrolidine, piperidine, morpholine, piperazine or N-methylpiperazine;
ring X is a phenyl ring or a ring of the formula III:-

[Formula III]

in which the dotted line is a double bond on one side of the nitrogen and Z is carbon or nitrogen such that ring X is a 5- or 6-membered heterocyclic aromatic ring which contains at least one nitrogen and may optionally contain one or two additional hetero atoms selected from oxygen, nitrogen and sulphur, ring X, where possible, carrying one or two optional substituents selected from fluorine, chlorine, bromine, 1-6C alkyl, 1-6C alkoxy, 1-6C alkylthio, trifluoromethyl, hydroxy and amino;

A is phenylene or 5-7C cycloalkylene, or a 1-8C alkylene chain which is optionally substituted by one or two 1-3C alkyls and into which is optionally inserted, as part of the backbone of the chain, one or two groups selected from oxygen, sulphur, NH, 1-6C N-alkyl, cis and trans vinylene, ethynylene, phenylene and 5-7C cycloalkylene, provided that the shortest link between ring X and C=D is of at least 3 atoms, provided that when an optional insertion is made in chain A which results in the inserted group being directly attached to C=D the inserted group is other than oxygen, sulphur, NH or N-alkyl, and provided that no two insertions selected from oxygen, sulphur, NH and N-alkyl are directly attached one to the other;

D is oxygen or sulphur;

$R^3$ is 1-6C alkyl, phenyl or benzyl;

and the pharmaceutically-acceptable acid addition salts thereof in association with a pharmaceutically-acceptable diluent or carrier.

2. A pharmaceutical composition as claimed in claim 1 wherein, in the guanidine derivative of the formula I, $R^1$ and $R^2$ are selected from the group consisting of hydrogen, 2,2,2-trifluoroethyl, 2,2,2-trichloroethyl, 2-chloro-2,2-difluoroethyl, 2,2-dichloro-2-fluoroethyl, 2-bromo-2,2-difluoroethyl, 2,2-dibromo-2-fluoroethyl, 2-fluoroethyl, 2-chloroethyl, 2,2-difluoroethyl, 2,2-dichloroethyl, 2-chloro-2-fluoroethyl, 2-bromo-2-fluoroethyl, 2,2,3,3-tetrafluoropropyl, 2,2,3,3,3-pentafluoropropyl, 1,1,1,3,3,3-hexafluoroisopropyl, 1,3-dichloro-1,1,3,3-tetrafluoroisopropyl, 1-chloro-1,1,3,3,3-pentafluoroisopropyl, 1,3-difluoroisopropyl, 2,2,3,3,4,4,4-heptafluorobutyl, 2,2,3,3-tetrafluorocyclopropyl, 2-chloro-2,3,3-trifluorocyclopropyl, 2,2-difluorocyclopropyl, 2-chloro-3,3-difluorocyclopropyl, 2,2,3,3,4,4-hexafluorocyclobutyl, 2-chloro-2,3,3,4,4-pentafluorocyclobutyl, (1,2,2,3,3-pentafluorocyclopropyl)methyl, (2-chloro-1,2,3,3-tetrafluorocyclopropyl)methyl, (1,2,2,3,3,4,4-heptafluorocyclobutyl)methyl, (2-chloro-1,2,3,3,4,4-hexafluorocyclobutyl)methyl, methyl, ethyl, propyl, isopropyl, butyl, cyclopropyl, cyclobutyl, cyclopropylmethyl and cyclobutylmethyl, provided at least one of $R^1$ and $R^2$ is a halogen-substituted radical,
or $-R^2$ is hydrogen and $-R^1$ is a radical of the formula II given in claim 1 in which W is an unbranched 2-6C alkylene chain which is optionally substituted by one or two methyls, E is oxygen, sulphur, sulphinyl or sulphonyl or a radical of the formula $NR^5$ in which $R^5$ is hydrogen or methyl, $R^4$ is hydrogen or methyl, or $R^4$ and $R^5$ are joined to form, together with the nitrogen to which they are attached, a pyrrolidine, piperidine, morpholine, piperazine or N-methylpiperazine;
ring X is a phenyl, oxazole, thiazole, imidazole, 1,2,4-thiadiazole, 1,2,4-oxadiazole, 1,2,3-triazole, 1,2,4-triazole, pyrazole, pyrazine, pyridine, pyrimidine or 1,3,5-triazine ring, ring X being optionally substituted, where possible, by one or two substituents selected from

fluorine, chlorine, bromine, methyl, methoxy, methylthio, trifluoromethyl, hydroxy and amino;

-A- is phenylene, cyclopentylene, cyclohexylene, trimethylene, tetramethylene, pentamethylene, thioethylene, thiotrimethylene, thiotetramethylene, thiopentamethylene, oxyethylene, oxytrimethylene, oxytetramethylene, methylenethiomethylene, methylenethioethylene, methylenethiopropylene, methyleneoxymethylene, methyleneoxyethylene, ethyleneoxyethylene, oxy-2-methylethylene, thiopropylenethiomethylene, oxyethyleneoxymethylene, iminoethylene, iminopropylene, vinylenepropylene, oxymethylenevinylene, 1,3-phenylene, 1,3-cyclopentylene, methylene-1,4-phenylene, ethyleneoxymethylene-1,4-phenylene, oxy-1,3-phenylenemethylene or thiomethyleneethynylenemethylene;

D is oxygen or sulphur; and

$R^3$ is methyl, ethyl, phenyl or benzyl.

3. A composition as claimed in claim 1 in which the compound of the formula I is methyl 5-[3-(2-[2,2,2-trifluoroethyl]guanidino)pyrazol-1-yl]valerate.

4. A composition as claimed in any of claims 1 to 3 which is in tablet or capsule form.

IJSB/YB : PH32413

27 Jun 83